# EUROPEAN PATENT SPECIFICATION

(11) **EP 2 039 365 B1**
(45) Date of publication and mention of the grant of the patent: **20.04.2016**
(21) Application number: 07737233.2
(22) Date of filing: 30.05.2007
(51) Int. Cl.: A61K 35/20, A23C 17/00, A23L 33/10, A61P 3/00, A61P 3/04

(54) **VISCERAL FAT ACCUMULATION INHIBITOR, AND AGENT FOR PROMOTING THE INCREASE IN AND/OR INHIBITING THE DECREASE IN BLOOD ADIPONECTIN LEVEL**
HEMMER DER ANSAMMLUNG VON VISZERALFETT UND MITTEL ZUR FÖRDERUNG DES ANSTIEGS UND/ODER ZUR UNTERDRÜCKUNG DER SENKUNG DES ADIPONECTIN-SPIEGELS IM BLUT
INHIBITEUR DE L'ACCUMULATION DE GRAISSE VISCÉRALE, ET AGENT POUR FAVORISER L'AUGMENTATION ET/OU INHIBER LA DIMINUTION DU TAUX D'ADIPONECTINE DANS LE SANG

(30) Priority: 31.05.2006 JP 2006152553
(43) Date of publication of application: 25.03.2009
(73) Proprietor: Megmilk Snow Brand Co., Ltd., Sapporo-shi Hokkaido 0650043 (JP)
(72) Inventor: KAWAKAMI, Hiroshi, Sapporo-shi, Hokkaido 065-0043 (JP); HIGURASHI, Satoshi, Sapporo-shi, Hokkaido 065-0043 (JP); MATSUYAMA, Hiroaki, Sapporo-shi, Hokkaido 065-0043 (JP)
(74) Representative: Gill Jennings & Every LLP
(86) International application number: PCT/JP2007/000577
(87) International publication number: WO 2007/138749

(56) References cited:
- WO-A1-2005/092367
- WO-A1-2005/094866
- WO-A1-2006/006286
- JP-A- 05 030 942
- JP-A- 2001 275 614
- MILAN GABRIELLA ET AL: "Resistin and adiponectin expression in visceral fat of obese rats: effect of weight loss." OBESITY RESEARCH NOV 2002 LNKD- PUBMED:12429872, vol. 10, no. 11, November 2002 (2002-11), pages 1095-1103, XP002588438 ISSN: 1071-7323
- ASAYAMA KOHTARO ET AL: "Decrease in serum adiponectin level due to obesity and visceral fat accumulation in children." OBESITY RESEARCH SEP 2003 LNKD- PUBMED:12972677, vol. 11, no. 9, September 2003 (2003-09), pages 1072-1079, XP002588439 ISSN: 1071-7323
- SPITSBERG V L: "Invited review: Bovine milk fat globule membrane as a potential nutraceutical" JOURNAL OF DAIRY SCIENCE, AMERICAN DAIRY SCIENCE ASSOCIATION, US LNKD- DOI:10.3168/JDS.S0022-0302(05)72906-4, vol. 88, no. 7, 1 July 2005 (2005-07-01), pages 2289-2294, XP002436135 ISSN: 0022-0302
- ARITA Y. ET AL.: 'Paradoxical Decrease of an Adipose-Specific Protein Adiponectin' BIOCHEMICAL AND BIOPHYSICAL RESEARCH COMMUNICATIONS vol. 257, 1999, pages 79 - 83, XP000867719
- LOUISE MENNEN ET AL: 'POSSIBLE PROTECTIVE EFFECT OF BREAD AND DAIRY PRODUCTS ON THE RISK OF THE METABOLIC SYNDROME' NUTRITION RESEARCH vol. 20, no. 3, 01 January 2000, pages 335 - 347, XP055061053 DOI: 10.1016/S0271-5317(00)00127-5
- PEREIRA ET AL: 'Dairy consumption, obesity, and the insulin resistance syndrome in young adults: the CARDIA Study.' JAMA vol. 287, no. 16, 01 April 2002, pages 2081 - 2089, XP055061056
- L AZADBAKHT ET AL: 'Dairy consumption is inversely associated with the prevalence of the metabolic syndrome in Tehranian adults' AM J CLIN NUTR vol. 82, no. 3, 01 January 2005, pages 523 - 530, XP055061102
- IQBAL NAYYAR: "The burden of type 2 diabetes: strategies to prevent or delay onset.", VASCULAR HEALTH AND RISK MANAGEMENT 2007, vol. 3, no. 4, 2007, pages 511-520, ISSN: 1176-6344

## Description

### TECHNICAL FIELD

The present invention relates to an agent for promoting the increase and/or inhibiting the decrease in a blood adiponectin level that contains a fat globule membrane component as an active ingredientThe agent for promoting the increase and/or inhibiting the decrease in blood adiponectin level according to the present invention can promote an increase in blood adiponectin level and/or inhibit a decrease in blood adiponectin level by ingestion. A decrease in blood adiponectin level is considered to be a cause of metabolic syndrome that relates to circulatory system diseases in future such as insulin resistance and glucose metabolic disorder. The present invention is effective for preventing and treating the metabolic syndrome.

### BACKGROUND ART

In recent years, the number of patients exhibiting disease conditions such as diabetes, hypertension, hyperlipemia, or arteriosclerosis that are lifestyle-related diseases has increased along with westernization of lifestyle. In particular, deaths due to cardiovascular disease and cerebrovascular disease are responsible for about one-third of all deaths, and the number of the deaths has increased year by year. Therefore, measures against cardiovascular disease and cerebrovascular disease have become important national issues. The risk of developing these arteriosclerotic diseases increases significantly due to an accumulation of risk factors such as hypertension, hyperlipemia, glucose intolerance and the like. The conditions accumulating of such risk factors have been widely recognized as metabolic syndrome. According to a study conducted on 120,000 Japanese corporate workers, it is reported that a person who has one of the risk factors of "obesity", "hypertension", "hyperglycemia", "hypertriglyceridemia", and "hypercholesterolemia" even in mild case has a five times higher risk of developing cardiac disease, a person who has two of the risk factors has a ten times higher risk of developing heart disease, and a person who has three or four of the risk factors has a 31 times higher risk of developing heart disease. The Ministry of Health, Labour and Welfare of Japan reported that the number of patients suffering from hypertension is 39,000,000, the number of patients suffering from hyperlipemia is 22,000,000, the number of patients suffering from diabetes (including pre-diabetes) is 16,200,000, and the number of patients suffering from obesity is 4,680,000. The number of patients suffering from these diseases has increased year by year.

The term "metabolic syndrome" refers to "multiple risk factor syndrome that is caused by visceral fat accumulation and complicated by multiple diseases such as insulin resistance, glucose metabolic disorder, lipid metabolism disorder, and hypertension based on said visceral fat accumulation and may exhibit pathological condition likely to get arteriosclerosis". Visceral fat accumulation is a just fundamental factor of the metabolic syndrome. Fat tissue which is the biggest secretory tissue in a living body produces various endocrine factors, and is involved in the maintenance of homeostasis of a living body. However, it becomes clear that excessive visceral fat accumulation breaks down the secretion balance between the endocrine factors and causes various pathological conditions. In particular, the endocrine factors such as plasminogen activator inhibitor (PAI-1), tumor necrosis factor (TNF-α), leptin and the like secrete increasing quantities thereof due to visceral fat accumulation so that thrombosis, insulin resistance, glucose metabolic disorder, hypertension, and the like occur.

On the other hand, adiponectin that is specifically secreted by a fat tissue is normally contained in blood at a high level. The adiponectin level is known to decrease due to visceral fat accumulation. Adiponectin is known to have various physiological functions such as antidiabetic action, anti-atherogenic action, anti-inflammatory effect, antihypertensive property and the like. It is very important to promote an increase of the adiponectin level in blood or suppress a decrease of the adiponectin level in blood in order to prevent or treat the metabolic syndrome.

Drug therapy has been employed as measures to treat each pathological condition involved in the metabolic syndrome. However, the issue of drug therapy is to require a prescription and bring side effects. Even if one pathological condition is treated, a serious pathological condition may obviously occur due to other pathological conditions. Therefore, it is necessary to regulate the balance between the endocrine factors derived from fat tissue which is present on the upstream of these situations. Accordingly, a change in lifestyle, for example, exercise therapy or diet therapy rather than drug therapy is considered to be important in order to prevent or treat the metabolic syndrome caused by visceral fat accumulation. Therefore, a food or drink that can be taken daily, has high safety even by intake over a long period of time, and is effective for preventing or treating the metabolic syndrome caused by visceral fat accumulation has been desired.

Pereira et al., "Dairy consumption, obesity, and the insulin resistance syndrome in young adults: the CARDIA study", JAMA, vol. 287, no. 16, 1 April 2002, pages 2081-2089 teaches that dietary patterns characterized by increased dairy consumption have a strong inverse association with IRS (insulin resistance syndrome = metabolic syndrome) among overweight adults and may reduce risk of type 2 diabetes and cardiovascular disease.

### DISCLOSURE OF THE INVENTION

### PROBLEMS TO BE SOLVED BY THE INVENTION

An object of the present invention is to provide an agent for use in the prevention or treatment of insulin resistance and glucose metabolic disorder, wherein the agent consists of a milk fat globule membrane component as an active ingredient which is purified from buttermilk and/or butter serum, and in the use, the agent promotes an increase in blood adiponectin level and/or inhibits a decrease in blood adiponectin level.

### MEANS FOR SOLVING THE PROBLEMS

The inventors of the present invention extensively searched for a component that reduces visceral fat that is considered to be the cause of the metabolic syndrome and a component that does not decrease blood adiponectin level that is considered to increase the risk of circulatory diseases if the level in blood decreases, from milk composition. As a result, the inventors found that a fat globule membrane component in milk fat has an extremely high visceral fat accumulation inhibition effect and blood adiponectin level increase promotion and/or decrease inhibition effect. This finding has led to the completion of the present invention.

The fat globule membrane component is a membrane that covers a milk fat globule secreted from a mammary gland. The fat globule membrane component has a function of dispersing fat in milk, and also has a number of physiological functions as a diet of newborn animals. The milk fat globule membrane of bovine milk comprises about 45% of proteins and about 55% of lipids. The milk fat globule membrane contains high-molecular-weight glycoproteins called milk mucin as proteins. The milk fat globule membrane contains about 70% of triacylglycerol, about 27% of phospholipids, about 3% of cholesterol, and the like as lipids. The milk fat globule membrane is known to have functions protecting the milk fat globule, stabilizing a milk fat emulsion, promoting lipid digestion, and preventing from infection of specific bacteria, for example.

The present invention provides an agent for promoting the increase in blood adiponectin level and/or inhibiting the decrease in blood adiponectin level that contains a milk fat globule membrane component as an active ingredient.

As the milk fat globule membrane component that may be used in the present invention, buttermilk obtained when producing butter grains by churning the cream which is obtained by centrifuging milk of a mammal such as cow, may be used as is. Alternatively, to the above-mentioned cream water in equal amounts is added and mixed, the mixture is centrifuged to prepare cream having the original fat percentage, these operations repeat several times to obtain cream removed fat-free milk components. Buttermilk obtained when producing butter grains by churning the cream removed fat-free milk components may be used as the milk fat globule membrane component. A butter serum obtained as a residue when heating and centrifuging the butter grains to produce butter oil may also be used as the milk fat globule membrane component.

The above-mentioned buttermilk and butter serum contain a sufficient amount of milk fat globule membrane component, and may be used as is as the milk fat globule membrane component. Alternatively, these buttermilk and butter serum may be used after further purifying by dialysis, ammonium sulfate fractionation, gel filtration, isoelectric precipitation, ion-exchange chromatography, solvent fractionation, ultrafiltration (UF), microfiltration (MF), or the like to increase the purity of the milk fat globule membrane component. For example, a composition having a high content of the fat globule membrane component may be obtained using separation/filtration technology by a UF membrane or MF membrane. The lipid fraction cannot pass through the UF membrane or MF membrane so that the proteins, lactose, and minerals can be removed. The molecular weight cut-off of the UF membrane and the pore size of the MF membrane may be not so rigorous, those skilled in the art thus can be set appropriate value based on experiments. For example, when using microfiltration, the pore size of the MF membrane may be 1.2 µm or less, and preferably 0.2 µm or less. When using ultrafiltration, the molecular weight cut-off of the UF membrane may be 10,000 or more, and preferably 50,000 to 100,000 or more as a tentative target. A concentrated liquid obtained by filtration may be spray-dried to prepare a composition having a high fat globule membrane component content. Alternatively, a concentrated liquid obtained by filtration using a UF membrane or fMF membrane may be homogenized (100 kg/cm² or more), subjected to microfiltration or ultrafiltration, and then spray-dried to prepare a composition having a higher fat globule membrane component content. The crude fraction of the fat globule membrane component fraction may be obtained as follows. Specifically, a composition concentrating the fat globule membrane component content is treated with a polar solvent such as ethanol or methanol or a mixture of a non-polar solvent and a polar solvent, for example, ether-ethanol (1:3 v/v), chloroform-methanol (2:1 v/v), or chloroform-methanol-water (1:2:0.8 v/v) to extract crude fat. The crude fat is then subjected to acetone fractionation to be able to obtain an acetone-insoluble fraction containing rich fat globule membrane component. Butter serum, which is a residue obtained when preparing butter oil from cream, contains a large amount of a fat globule membrane component, and is one of the preferable raw materials. Crude fat is extracted from the butter serum using ethanol, and acetone fractionation of the crude fat may be used. Alternatively, a fraction extracted with a hexane-ethanol-water mixed solvent may also be used (JP-A-7-173182). Alternatively, a fat globule membrane component derived from commercially available milk may be used.

### EFFECT OF THE INVENTION

The agents that are given blood adiponectin level increase promotion effect and/or decrease inhibition effect according to the present invention are effective for preventing or treating the metabolic syndrome that is considered to be caused by a decrease in blood adiponectin level. Since the agents that are given blood adiponectin level increase promotion and/or decrease inhibition effect according to the present invention utilize the milk fat globule membrane component, a large amount of products can be supplied at relatively low cost. Moreover, these products have a feature of very high safety.

### BEST MODE FOR CARRYING OUT THE INVENTION

The agent for promoting the increase and/or inhibiting the decrease in blood adiponectin level according to the present invention contains the milk fat globule membrane component as an active ingredient. The dosage form of the agent for promoting the increase and/or inhibiting the decrease in blood adiponectin level may include a tablet, a capsule, a granule, a powder, or the like. The agent resulting in a blood adiponectin level increase promotion and/or decrease inhibition effect may be mixed into food or drink products, and for example may include products obtained by mixing the milk fat globule membrane component into food or drink such as milk, milk-based drink, coffee beverage, juice, jelly, cookie, bread, noodle, sausage and the like, various dried milks, and a nutrient composition for infants, young children, low birth weight infants, and the like. Since the food or drink can be taken daily and has a blood adiponectin level increase promotion and/or decrease inhibition effect, such a product is effective for preventing or treating the metabolic syndrome caused by a decrease in blood adiponectin level.

In the present invention, in order to exhibit a blood adiponectin level increase promotion and/or decrease inhibition effect, the formulation amount of the milk fat globule membrane component may be adjusted so that the milk fat globule membrane component may be ingested 0.1 to 5000 mg per day in the case of adult.

The present invention is described in more detail below by way of examples and test examples. Note that the following examples should not be construed as limiting the present invention.

### Example 1

Cream of which the fat percentage was adjusted to 40% was churned to separate butter grains and buttermilk. The buttermilk was then freeze-dried to obtain a milk fat globule membrane component.

### Example 2

Cream of which the fat percentage was adjusted to 40% and water were mixed in equal amounts, and the mixture was separated using a separator to prepare cream having a fat percentage of 40%. These operations were repeated three times to remove fat-free milk components from the cream. The resulting cream was then churned to separate butter grains and buttermilk. The obtained buttermilk was then freeze-dried to obtain a concentrated milk fat globule membrane component.

### Example 3

Cream of which the fat percentage was adjusted to 40% and water were mixed in equal amounts, and the mixture was separated using a separator to prepare cream having a fat percentage of 40%. These operations were repeated three times to remove fat-free milk components from the cream. The resulting cream was then churned to separate butter grains and buttermilk. The obtained buttermilk was treated overnight with a 50% saturated ammonium sulfate solution. The supernatant was then collected by centrifugation. After dialyzing the supernatant using water, the resulting product was freeze-dried to obtain a highly concentrated milk fat globule membrane component.

### [Test Example 1]

### Examination on blood adiponectin level increase promotion effect and/or decrease inhibition effect

A blood adiponectin level increase promotion and/or decrease inhibition effect was examined using the fat globule membrane components obtained in Examples 1, 2, and 3. An animal experiment was conducted on groups of eight animals. A high-fat feed formulating the fat globule membrane component obtained in Example 1, 2, or 3 was fed to three groups (fat globule membrane component feed groups 1 to 3), and a high-fat feed containing no fat globule membrane component was fed to another group (high-fat feed group). The high-fat feed was prepared using a milk casein as a protein source and butter oil as a lipid source. In the animal experiment, the feed was fed to each group for four weeks after breeding, and the high-fat feed or the feed containing the fat globule membrane component was then fed to the corresponding five groups for further four weeks, respectively. Blood was collected at the fourth week and the eighth week. The blood adiponectin level was measured using a mouse/rat adiponectin ELISA kit (manufactured by Otsuka Pharmaceutical Co., Ltd.).

The results are shown in Table 1. According to Table 1, it was found that while the blood adiponectin level of the high-fat feed group decreased with the passage of time, the blood adiponectin levels of the fat globule membrane component feed groups 1 to 3 increased with the passage of time. Specifically, it was confirmed that an increase in blood adiponectin level was promoted or a decrease in blood adiponectin level was suppressed by ingestion of the fat globule membrane component.

**[Table 1]**

| Feed group | Blood adiponectin level (µg/ml) | | Change rate (%) |
|---|---|---|---|
| | Four weeks | Eight weeks | |
| High-fat feed | 11.54 | 11.07 | 95.93 |
| Fat globule membrane component (Example 1) feed 1 | 11.32 | 13.74 | 121.38 |
| Fat globule membrane component (Example 2) feed 2 | 11.25 | 14.11 | 125.42 |
| Fat globule membrane component (Example 3) feed 3 | 10.67 | 14.82 | 138.89 |

### [Reference Test Example 1]

### Examination on visceral fat accumulation inhibition effect

A visceral fat accumulation inhibition effect was examined using the fat globule membrane components obtained in Examples 1, 2, and 3. An animal experiment was conducted on groups of eight animals. A high-fat feed formulating the fat globule membrane component was fed to three groups (fat globule membrane component feed groups 1 to 3), and a high-fat feed containing no fat globule membrane component was fed to another group (high-fat feed group). In the animal experiment, the high-fat feed was fed to each group for four weeks after breeding, and the high-fat feed or the feed containing the fat globule membrane component was then fed to the corresponding five groups for further four weeks, respectively. The animals were dissected in the eighth week , and the amount of visceral fat (mesentery, circumference of testicle, circumference of kidney, and posterior abdominal wall) was measured.

The results are shown in Table 2. According to Table 2, it was found that the amount of visceral fat of the fat globule membrane component feed group was generally lower than that of the high-fat feed group. Specifically, it was confirmed that visceral fat accumulation was suppressed by ingestion of the fat globule membrane component.

**[Table 2]**

| Feed group | Amount of visceral fat (g) | | | | Total amount of visceral fat (g) |
|---|---|---|---|---|---|
| | Mesentery | Circumference of testicle | Circumference of kidney | Posterior abdominal wall | |
| High-fat food | 2.96 | 3.68 | 0.98 | 2.56 | 10.18 |
| Fat globule membrane component (Example 1) feed 1 | 2.61 | 3.23 | 0.78 | 2.67 | 9.29 |
| Fat globule membrane component (Example 2) feed 2 | 2.58 | 3.36 | 0.85 | 2.34 | 9.13 |
| Fat globule membrane component (Example 3) feed 3 | 2.31 | 3.18 | 0.67 | 2.17 | 8.33 |

### Example 4

Raw materials were mixed according to the formulation shown in Table 3 and then granulated. A capsule was charged with the granulated product to obtain a capsule provided with a visceral fat accumulation inhibition effect or a blood adiponectin level increase promotion and/or decrease inhibition effect.

**[Table 3]**

| | |
|---|---|
| Fat globule membrane component (Example 3) | 20.0 wt% |
| Lactose | 24.5 wt% |
| Soluble starch | 55.0 wt% |
| Magnesium stearate | 0.5 wt% |

### Example 5

Raw materials were mixed according to the formulation shown in Table 4.
After charging a container with the mixture, the mixture was sterilized by heating to obtain a visceral fat accumulation inhibition drink or a blood adiponectin level increase promotion and/or decrease inhibition drink.

**[Table 4]**

| | |
|---|---|
| Fat globule membrane component (Example 3) | 2.5 wt% |
| Sugar | 7.5 wt% |
| Citric acid | 0.6 wt% |
| Apple juice | 10.0 wt% |
| Water | 79.4 wt% |

## Claims

1. An agent for use in the prevention or treatment of insulin resistance and glucose metabolic disorder, wherein the agent consists of a milk fat globule membrane component as an active ingredient which is purified from buttermilk and/or butter serum, and in the use, the agent promotes an increase in blood adiponectin level and/or inhibits a decrease in blood adiponectin level.

2. An agent for use according to claim 1 wherein the fat globule membrane component is a membrane that covers a milk fat globule secreted from a mammary gland.

## Patentansprüche

1. Ein Mittel zur Verwendung bei der Prävention oder Behandlung von Insulinresistenz und Glucosestoffwechselstörung, wobei das Mittel aus einer Milchfettkügelchenmembran-Komponente, die aus Buttermilch und/oder Butterserum aufgereinigt ist, als dem arzneilich wirksamen Bestandteil besteht und das Mittel im Gebrauch eine Erhöhung des Blut-Adiponectinspiegels fördert und/oder eine Senkung des Blut-Adiponectinspiegels hemmt.

2. Ein Mittel zur Verwendung gemäß Anspruch 1, wobei die Milchfettkügelchenmembran-Komponente eine Membran ist, die ein aus einer Milchdrüse abgeschiedenes Milchfettkügelchen bedeckt.

## Revendications

1. Agent destiné à être utilisé dans la prévention ou le traitement de la résistance à l'insuline ou d'un trouble du métabolisme du glucose, dans lequel l'agent comprend un composant de membrane de globule gras du lait comme un ingrédient actif qui est purifié à partir de babeurre et/ou de lactosérum, et dans l'utilisation, l'agent favorise une augmentation du taux sanguin d'adiponectine et/ou inhibe une diminution du taux sanguin d'adiponectine.

2. Agent destiné à une utilisation selon la revendication 1, dans lequel le composant de membrane de globule gras est une membrane qui recouvre un globule gras de lait sécrété à partir d'une glande mammaire.
